# EUROPEAN PATENT SPECIFICATION

(11) **EP 3 444 321 B1**
(45) Date of publication and mention of the grant of the patent: **22.05.2024**
(21) Application number: 18796814.4
(22) Date of filing: 16.05.2018
(51) Int. Cl.: C10L 3/00, B09B 3/00, C02F 11/08, B09B 5/00, C12P 5/02, A01C 3/02, C12M 1/107, C12M 1/00

(54) **METHOD FOR PRODUCING FUEL AND DEVICE FOR PRODUCING FUEL**
VERFAHREN ZUR HERSTELLUNG EINES KRAFTSTOFFES UND VORRICHTUNG ZUR HERSTELLUNG VON KRAFTSTOFF
PROCÉDÉ DE PRODUCTION D'UN COMBUSTIBLE ET DISPOSITIF DE PRODUCTION D'UN COMBUSTIBLE

(30) Priority: 01.06.2017 JP 2017108904
(43) Date of publication of application: 20.02.2019
(73) Proprietor: Takase Tech Co., Ltd., Kawasaki-shi, Kanagawa 210-0007 (JP)
(72) Inventor: TAKASE, Joji, Yokohama-shi Kanagawa 230-0077 (JP)
(74) Representative: Vossius & Partner Patentanwälte Rechtsanwälte mbB
(86) International application number: PCT/JP2018/018878
(87) International publication number: WO 2018/221215

(56) References cited:
- WO-A1-2008/128466
- CN-A- 104 511 031
- JP-A- 2006 028 272
- JP-A- 2007 203 213
- JP-A- 2009 119 378
- JP-A- 2016 215 145
- JP-B1- 3 613 567

## Description

### BACKGROUND OF THE INVENTION

### Field of the Invention

The present invention relates to a fuel production method and fuel production apparatus, particularly to a fuel production method and fuel production apparatus for conversion treatment of livestock excrement into appropriate properties for fuel for gasification power generation.

### BACKGROUND ART

For effective energy recovery from wastes such as biomass, gasification power generation technology has been proposed with the use of which electric power is generated using thermally decomposed gasses as a heat source obtained by thermal decomposition of wastes, as disclosed in a Patent Literature 1. In this technology, wastes charged in a gasification furnace are gasified by heating with supplying a gasification agent composed of oxygen and water. When the wastes for fuels are highly wetting material such as garbage or sewage sludge, decomposition is quite difficult due to high water content more than 80% and arises a problem where thermally decomposed gas cannot be sufficiently generated.

In Patent Literature 2, the present inventor has proposed a fuel production method to produce fuel using low-molecularization process. In this method, garbage is charged with supplying high-pressure steam in a reaction vessel and the condition inside of the vessel is kept at a pressure ranging from 1.50 MPa to 1.96 MPa, at a temperature ranging from 185 °C to 215 °C for 30 to 50 minutes, for separation of bonded molecules. This method enables to obtain methane gas usable for power generation through fermentation of the fuel.

Patent Literature 3 relates to cecropin antibacterial peptide fermentation sterilization equipment and a sterilization method thereof. Patent Literature 4 relates to a process for fuel production and a fuel production apparatus which are suitable for use in converting and treating wastes with a high water content such as garbage, sludge, remainder of fish, dung and urine for use as a fuel for gasification power generation.

### Citation List

### Patent Literature

Patent Literature 1: Japanese Unexamined Patent Application Publication No. 2016-121253
Patent Literature 2: Japanese Patent No. 3613567
Patent Literature 3: CN 104 511 031 A
Patent Literature 4: JP 3 613 567 B1

### SUMMARY OF INVENTION

### Technical Problem

Technology for hydrothermal reaction treatment is described in the Patent Literature 1. It deals with solid waste which is difficult to use for gasification generation fuel due to high water content of garbage or sewage sludge. The waste is converted to reduce the water content to solid fuel with appropriate water content by hydrothermal reaction treatment; that is, the fuel described in the Patent Literature 1 is a solid body.

In case of livestock excrement, arises another technical problem different from the case of garbage and sewage sludge. Livestock excrement, however, as a waste to be converted to fuel is difficult to solidify the fuel due to high water content, and solidification requires additional energy and is uneconomical.

In addition, ammonia generated from the excrement inhibits fermentation and this causes a problem of preventing smooth production of methane gas.

The excrement contains feed as digested; organic substances such as hydrocarbons, proteins, fats. Feed as digested is not easily decomposed into low-molecular weight organic compounds such as a monosaccharide, amino acids, and higher fatty acids, and thus it is difficult to transfer the excrement to a methane fermentation process site and to perform high-speed fermentation. A stench of ammonia from the excrement is also problematic.

These technical problems prevent commercial methane fermentation using livestock excrement as fuel.

Earnest research to solve the technical problems leads to complete the present invention; the research enables elimination of ammonia that inhibits methane fermentation from the livestock excrement and increase of the speed of methane fermentation and the amount of generated gases by decomposition into low-molecular weight substances of the feed as digested.

Research is conducted subject to methane fermentation and hydrothermal reaction for conversion treatment to fuel of livestock excrement. The livestock excrement and water (tap water) are put into a reaction vessel, and steam is further added. An attempt is done to intensify molecular motion by colliding the liquid containing the livestock excrement against stirring blades. As a result it is found that the livestock excrement become in low molecular state, saccharified, aminated and changes in ionic product occur. It is also found that the speed of methane fermentation and the amount of generated gases increase by a fact that the livestock excrement become in low molecular state, saccharified, aminated and generated ammonia as an inhibiter reduces.

The present invention is made in view of the above situation. The invention aims to provide a fuel production method and an apparatus thereof where ammonia that inhibits methane fermentation of livestock excrement is removed, and an increase of methane fermentation rate and an increase of gas generation amount can be achieved by low-molecularizing organic compounds such as hydrocarbons, proteins, and fats contained in the livestock excrement, and by decomposing them into compounds having low molecular weight.

### Solution to Problem

A fuel production method according to the present invention to achieve the aim includes a step of charging livestock excrement into a reaction furnace and charging water the amount of which is equal to or more than five (5) times or equal to or less than twenty (20) times as much as the charged livestock excrement, a step of injecting saturated steam at a pressure of 0.62 MPa or higher or 1.9 MPa or lower into the reaction vessel after the excrement and water are charged, a step of decomposing the excrement, with injecting steam, by starting stirring the content in the reaction vessel, removing ammonia, and low-molecularizing the organic bonded molecules, and a step of stopping stirring when the temperature of a bottom portion in the reaction vessel rises to 125 °C or above.

In the present invention, as described above, corresponding to the amount of the charged excrement, after a predetermined amount of water is put in and saturated steam of a predetermined pressure is additionally injected in the reaction vessel, with keeping on injecting the steam, stirring the content in the reaction vessel is started and continued until the temperature at the bottom of the reaction vessel rises up to a predetermined temperature, and is stopped when the temperature has risen above the predetermined temperature.

As described above, with charging the predetermined amount of water and injecting saturated steam at the predetermined pressure and stirring the content of the reaction vessel until the temperature at the bottom of the reaction vessel, ammonia is removed and the organic bonded molecules contained in the excrement are separated and decomposed. That is, the ammonia as an inhibitor is removed from the fuel made from excrement and decomposition into low-molecular weight organic compounds, saccharification, amination, and changes in ion product of the fuel can be achieved. As a result, using the fuel, high-speed fermentation of methane in the fermenter can be performed and increase in speed of methane fermentation and increase of generated gases can be achieved. Here, the excrement refers to chicken droppings, pig excrement, bovine dung, dung of horses and sheep, and the like.

The livestock excrement requires a large value of chemical oxygen demand measured with potassium dichromate (COD_{Cr}) and accordingly fermentation takes a longer time and economical loss increases. To enhance the efficiency of fermentation, it is necessary to put a predetermined amount of water in to dilute the livestock excrement with water to reduce the value of COD_{Cr}. On one hand, when the predetermined amount of water to be put is less than five (5) times as much as the charged excrement, the total organic carbon (TOC) against the COD value is larger and the fermentation speed becomes low, which is not preferable. On the other hand, when the predetermined amount of water is more than twenty (20) times as much as the charged excrement, due to the decreased TOC value the fermentation speed becomes slow and this causes to lose economic efficiency.

Injection of saturated steam at a predetermined pressure into the reaction vessel causes a hydrothermal reaction. When the pressure is less than 0.62 MPa, residual ammonia is observed and the efficiency of fermentation is not satisfactory. When the predetermined pressure is higher than 1.9 MPa, a carbonization phenomenon of organic compounds occurs when reaction product is discharged after the end of the reaction.

Stirring of the content in the reaction vessel is started with keeping on injecting the steam and stopped when the temperature at the bottom of the reaction vessel rises above 125 °C; thus stirring is stopped at a time the temperature rises above 125 °C because decomposition into low-molecular weight organic compounds, amination, and saccharification of the livestock excrement have already finished.

The stirring is preferably started at a time when the temperature at the bottom of the reaction vessel reaches 100 °C. Because the temperature of the liquid containing livestock excrement is lower at the bottom portion of the reaction vessel, by letting the temperature at the bottom of the reaction vessel be a reference and measuring the temperature thereof, the temperature of the whole organic compounds in the liquid can be confirmed to be higher than 100 °C. The reason why stirring is started at a time when the temperature at the bottom of the reaction vessel has reached 100 °C is that bonded molecules of the organic compounds contained in the excrement can be separated and decomposed; stirring is started to enhance the motion of molecules by colliding to the blades of the stirring means.

When the livestock excrement is chicken droppings, a fuel production method preferably includes a step of charging chicken droppings into a reaction furnace and charging water the amount of which is equal to or more than five (5) times or equal to or less than ten (10) times as much as the charged chicken droppings, a step of injecting saturated steam at a pressure 0.69 MPa or higher or 0.84 MPa or lower into the reaction vessel after the chicken droppings and water are charged, a step of decomposing the chicken droppings, with keeping on injecting the steam, by starting stirring the content in the reaction vessel, removing ammonia, and decomposing the organic bonded molecules, and a step of stopping stirring when the temperature of the bottom portion in the reaction vessel reaches a temperature in a range of 135 °C to 140 °C.

Namely, it is preferable for the case of chicken droppings that the inputted amount of water is equal to or more than five (5) times or equal to or less than ten (10) times as much as the charged chicken droppings, the pressure of the injected steam into the reaction vessel is equal to or more than 0.69 MPa or equal to or less than 0.84 MPa, and stirring is stopped at a time when the temperature at the bottom of the reaction vessel reaches a temperature in a range of 135 °C to 140 °C.

In a case where the livestock excrement is pig excrement, a fuel production method preferably includes a step of charging pig excrement into a reaction furnace and charging water the amount of which is equal to or more than ten (10) times or equal to or less than twelve (12) times as much as the charged pig excrement, a step of injecting saturated steam at a pressure 0.76 MPa or higher or 0.85 MPa or lower into the reaction vessel after the pig excrement and water are charged, a step of decomposing the pig excrement, with keeping on injecting the steam, by starting stirring the content in the reaction vessel, removing ammonia, and decomposing the organic bonded molecules, and a step of stopping stirring when the temperature of the bottom portion in the reaction vessel reaches a temperature in a range of 135 °C to 140 °C.

Namely, it is preferable for the case of pig excrement that the inputted amount of water is equal to or more than ten (10) times or equal to or less than twelve (12) times as much as the charged pig excrement, the pressure of the injected steam into the reaction vessel is equal to or more than 0.76 MPa or equal to or less than 0.85 MPa, and stirring is stopped at a time when the temperature at the bottom of the reaction vessel reaches a temperature in a range of 135 °C to 140 °C.

A fuel production apparatus according to the present invention to achieve the aim described above includes a reaction vessel having stirring means, steam injection means for injecting saturated high-pressure steam into the reaction vessel in which livestock excrement is charged, a temperature sensor which measures the temperature at a bottom portion of the reaction vessel, and control means that controls injection of the saturated steam using the steam injection means to keep the pressure of the reaction vessel in a range of 0.62 MPa to 1.9 MPa, controls to start operation of the stirring means at a time when the temperature at the bottom of the reaction vessel passes 100 °C, and controls to stop operation of the stirring means at a time when the temperature at the bottom reaches 125 °C.

When the livestock excrement is chicken droppings, the apparatus preferably includes the control means that controls injection of the saturated steam using the steam injection means to keep the pressure of the reaction vessel in a range of 0.69 MPa to 0.84 MPa, controls to start operation of the stirring means at a time when the temperature at the bottom of the reaction vessel passes 100 °C, and controls to stop operation of the stirring means at a time when the temperature at the bottom reaches a temperature in a range of 135 °C to 140 °C.

When the livestock excrement is pig excrement, the apparatus preferably includes the control means that controls injection of the saturated steam using the steam injection means to keep the pressure of the reaction vessel in a range of 0.76 MPa to 0.85 MPa, controls to start operation of the stirring means at a time when the temperature at the bottom of the reaction vessel passes 100 °C, and controls to stop operation of the stirring means at a time when the temperature at the bottom reaches a temperature in a range of 135 °C to 140 °C.

### Advantageous Effects of Invention

The present invention enables to obtain a fuel production method and a fuel production apparatus where ammonia that inhibits methane fermentation of livestock excrement is removed, and an increase of methane fermentation rate and an increase of gas generation amount can be achieved by decomposing organic compounds such as hydrocarbons, proteins, and fats contained in the livestock excrement, and by decomposing them into compounds having low molecular weight.

### BRIEF DESCRIPTION OF DRAWINGS

FIG. 1 is a flowchart of steps from collection of livestock excrement through methane gasification, showing a fuel production method according to embodiments of the invention.
FIG. 2 is a schematic diagram showing a production apparatus.

### DESCRIPTION OF THE PREFERRED EMBODIMENTS

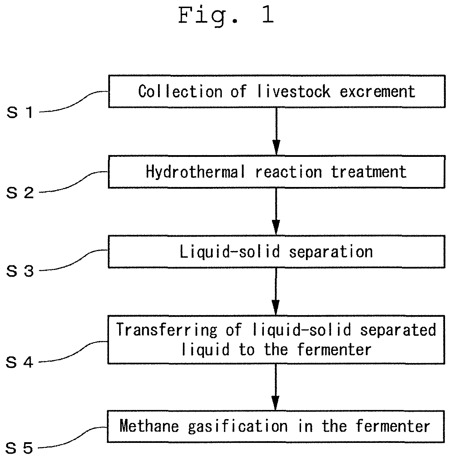

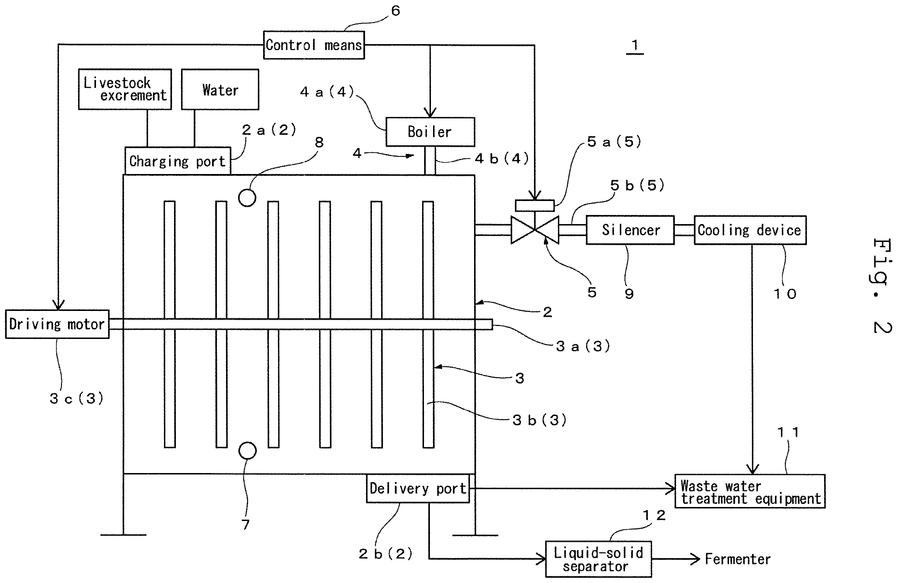

A fuel production method according to the present invention will be explained in detail with reference to FIGS. 1 and 2. In the present fuel production method, a hydrothermal reaction treatment (S2) is performed to collected livestock excrement (S1), as shown in FIG. 1. In the present fuel production method, collected livestock excrement (S1) is subjected to hydrothermal reaction treatment (S2), as shown in Fig.1. A characteristic aspect of this method is in that the hydrothermal reaction treatment is performed under a predetermined condition. The livestock excrement charged in a reaction vessel with putting a predetermined amount of water and injecting saturated steam at a predetermined pressure is stirred until temperature of a bottom portion of the reaction vessel reaches a predetermined temperature; this operation suppresses ammonia generation and low-molecularizes by decomposing bonded compounds of organic matters contained in the excrement, and produces fuel that is decomposed into low-molecular weight compounds, aminated and saccharified. Detailed condition of the hydrothermal reaction treatment will be described later.

The liquid and solid matters subjected to the hydrothermal reaction are separated into an organic-matter containing liquid that contains (simply referred to a liquid) fermented components of livestock excrement, decomposed low-molecular organic compounds, and solid matters (S3), using a solid-liquid separator. A publicly known apparatus can be employed as a solid-liquid separator. The separated organic-matter containing liquid is transferred to a fermenter (S4) and stored in the fermenter. A publicly known transferring means can be also usable for transferring the organic-matter containing liquid. The organic-matter containing liquid in the fermenter is kept at a predetermined temperature to perform fermentation treatment with methane bacteria; that is, methane gas production. This treatment can be performed similarly to the conventional method.

As described above, the present invention, directed to livestock excrement, aims at producing fuel from organic-matter containing liquid that contains organic compounds in livestock excrement, using processes in which an appropriate amount of water is added to livestock excrement and hydrothermal reaction treatment is performed under a predetermined condition.

An embodiment of a fuel production apparatus according to the present invention is described with reference to Fig. 2. As shown in Fig. 2, a fuel production apparatus 1 includes a reaction vessel 2 in which livestock excrement is stored and treated, a charging port 2a for charging the livestock excrement and water (tap water) into the reaction vessel 2, and a stirring means 3 for stirring a mixed liquid of the livestock and the water both charged into the reaction vessel. The fuel production apparatus 1 includes a steam injection means 4 for injecting high-pressure steam to the mixed liquid of livestock and water in the reaction vessel 2, a pressure adjusting means 5 for adjusting the pressure in the reaction vessel 2, and a control means 6 for control the stirring means 3, the steam injection means 4 and the pressure adjusting means 5.

Detailed explanation on each of the constitution sections will be given below. The reaction vessel 2 is composed of a pressure-resistant vessel in which the mixed liquid of livestock excrement and water is treated. A feeding port 2a is provided at the top of the reaction vessel and a delivery port 2b is provided at the bottom for leading the liquid containing low molecular weight organic compounds that is obtained by hydrothermal reaction treatment. The feeding port 2a and delivery port 2b have sealed structure with packings resistant to high temperature and high pressure of the reaction vessel when the liquid is subjected to hydrothermal treatment. The feeding port 2a and delivery port 2b are also provided with a system control function not to respond open/close operation unless the pressure comes down below 0.015 MPa for the sake of safety.

A temperature sensor 7 is provided at the bottom of the reaction vessel and a pressure sensor 8 is provided at the inner top of the vessel to respectively detect the temperature and the pressure inside of the vessel 2. In particular, the temperature sensor 7, provided at the bottom of the vessel, can measure the temperature of the organic-matters containing liquid at the bottom of the vessel; lower part of the liquid has a relatively lower temperature. Namely, it is possible to confirm that the temperature of the entire organic-matter containing liquid is higher than the temperature measured by the temperature sensor 7, by measuring the temperature of the lower part of the liquid located at the bottom of the reaction vessel 2.

The stirring means 3 is provided for making the pressure and temperature of the liquid uniform over the entire liquid. The stirring means 3 is extended longitudinally in the reaction vessel 2 and is provided with a rotatably-journaled horizontal rotary shaft 3a and a stirring blade 3b attached to the horizontal rotary shaft 3a. A driving motor 3c is coupled to the horizontal rotary shaft 3a and drives the stirring blade 3b.

The steam injection means 4 includes a boiler 4a to generate high-pressure steam, saturated steam, and a steam supply pipe 4b for feeding the steam generated by the boiler 4a, saturated steam, into the reaction vessel 2. The pressure of the steam, saturated steam, generated by the boiler 4a is maintained to be constant, and the pressure in the reaction vessel 2 is adjusted by injection amount of the high pressure steam, saturated steam. The pressure of the high-pressure steam, high-pressure saturated steam, determines the temperature inside the reaction vessel 2 and the interior of the reaction vessel 2 is kept at a high temperature. The steam supply pipe 4b is disposed above the horizontal rotary shaft 3a and connected to the reaction vessel 2 in the substantially horizontal direction.

The pressure adjusting means 5 includes a pressure adjusting valve 5a that is electrically controlled to achieve an open/close control, and an exhaust pipe 5b for exhausting the steam in the reaction vessel 2 through the pressure adjusting valve 5a. When the pressure inside the reaction vessel 2 exceeds a predetermined value, the pressure adjusting valve 5a is released to depressurize the reaction vessel 2 to maintain the pressure to be the predetermined value. In addition, a cooling device 10 is connected to the exhaust pipe 5b through a silencer 9, and cools and liquefy the steam from the reaction vessel 2 to drain to waste water treatment equipment 11. It is designed, with the use of the silencer 9, to enables to meet the requirement of noise control regulations and to install the fuel production apparatus in an urban district.

The control means 6 is electrically connected to the stirring means 3, steam injecting means 4 and pressure adjusting means 5, and controls them. The control means6 controls revolution direction and revolution speed of the driving motor 3c, and also controls to start and stop of stirring of the liquid in the reaction vessel 2. The control means 6 further controls the injection amount of steam injected by the steam injecting means 4 so as to keep the pressure at a predetermined value for a predetermined time at which pressure the liquid in the reaction vessel 2 is converted to have appropriate properties for gasification power generation.

The control means 6 is electrically connected to the temperature sensor 7 and the pressure sensor 8 both in the reaction vessel and controls to start and stop the driving motor 3c by receiving a signal from the temperature sensor 7. The control means 6 performs a feedback control to keep the temperature and pressure respectively at a predetermined value on the basis of the detection results of the temperature sensor 7 and the pressure sensor 8 in the reaction vessel 2. Specifically, when the temperature or the pressure in the reaction vessel 2 decreases, the control means 6 controls to increase the temperature and pressure by increasing the injection amount of the high-pressure steam from the steam injection means 4. On the contrary, when the temperature or the pressure in the reaction vessel 2 rises above the predetermined values, the control means 6 controls to lower the temperature and pressure by discharging the high-pressure steam by releasing the pressure adjusting valve 51 of the pressure adjusting means 5.

A fuel production method using the fuel production apparatus according to the present invention will be explained. Livestock excrement is charged into the reaction vessel through the charging port 2a and a predetermined amount of water (tap water) is also charged corresponding to the amount of charged livestock excrement.

The reason of charging the predetermined amount of water into the reaction vessel is to enhance fermentation efficiency by diluting the concentration of the organic components, because of high CODcr for the livestock excrement, longer time necessary for fermentation, and large loss in commercial economy. When the predetermined amount of water is less than five (5) times as much as the amount of the charged livestock excrement, the total organic carbon (TOC) against the COD value is higher and the fermentation speed becomes low; this is not preferable. On the contrary, when the predetermined amount of water is more than twenty (20) times as much as the charged excrement, the decreased TOC value causes lack of nutrition necessary for alternation of generation of methane bacteria, and consequently the fermentation speed becomes slow and this causes to lose economic efficiency; this is also not preferable.

It is not preferable that a large amount of water inhibits separation of organic bonded molecules and causes to low-molecularize lower-molecular eight organic compounds contained in the livestock excrement, amination, and saccharization difficult. Therefore, it is desirable to charge water into the reaction vessel 2 equal to or more than five (5) times or equal to or less than twenty times (20) as much as the charged livestock excrement.

In the case of chicken droppings as the livestock excrement, it is desirable to charge water into the reaction vessel 2 equal to or more than five (5) times or equal to or less than twenty times (20) as much as the charged livestock excrement, but particularly desirable to charge water into the reaction vessel 2 equal to or more than five (5) times or equal to or less than ten times (10) as much as the charged chicken droppings. In case of pig excrement as the livestock excrement, it is desirable to charge water into the reaction vessel 2 equal to or more than five (5) times or equal to or less than twenty times (20) as much as the charged livestock excrement, but particularly desirable to charge water into the reaction vessel 2 equal to or more than five (5) times or equal to or less than twelve times (12) as much as the charged pig excrement.

A pressure value in the reaction vessel during processing according to the amount of charged livestock excrement and a temperature values in the reaction vessel according to the pressure value are set in the control means 6 in advance. In addition, temperatures at which stirring is started and stopped are set in the control means 6 in advance.

The pressure to be set is a necessary pressure to convert the livestock excrement into fuel appropriate for gasification power generation.

High pressure saturated steam is supplied in the reaction vessel 2 through the steam supply pipe 4b disposed above the horizontal rotary shaft 3a. The temperature inside the reaction vessel is set to a temperature determined by a theoretical pressure of the saturated steam.

The pressure inside the reaction vessel is set from 0.62 MPa to 1.92 MPa. When the pressure in the reaction vessel is less than 0.62 MPa, it is not preferable that ammonia remains and the efficiency of fermentation is low. On the contrary, when the pressure exceeds 1.9 MPa, it is not preferable because carbonization of organic matters in the liquid proceeds and generation of methane gas takes longer time, therefore large economic effects is not expected. In the case of chicken droppings, the pressure in the reaction vessel is desirably kept between 0.62 MPa and 1.9 MPa, and more preferably in a range of 0.69 MPa to 0.84 MPa. In the case of pig excretion, the pressure is desirably kept between 0.62 MPa and 1.9 MPa, more preferably in a range of 0.76 MPa to 0.85 MPa.

Stirring in the reaction vessel is started, while injecting steam on, when the temperature of the bottom part of the reaction vessel reaches 100 °C and stopped when the temperature thereof exceeds 125 °C. The reason why the stirring is started when the temperature reaches 100 °C is that as the temperature of the organic compounds contained in the liquid also reaches 100 °C, stirring makes decomposition at a molecular level becomes easier and activates molecular motion by colliding organic compounds against the stirring blades. The reason why the stirring is stopped when the temperature exceeds 125 °C that at this temperature organic compounds contained in the charged liquid have already been saccharized, aminated and decomposed into low-molecular weight compounds.

It is desirable to start stirring when the temperature of the bottom of the reaction vessel reaches 100 °C and to stop stirring when the temperature exceed 125 °C for the case the livestock excrement is chicken droppings or hog dunk; more preferably stirring is started at 100 °C and finished when the temperature rises up in a range of 135 °C to 140 °C.

The control means 6 controls the steam injecting means 4 and pressure adjusting means 5 to maintain the temperature and the pressure in the reaction vessel 2 at a predetermined temperature and pressure on the basis of the detection results of the temperature sensor 7 and the pressure sensor 6, respectively.

The control means 6 stops stirring when the temperature of the bottom of the reaction vessel reaches the predetermined temperature, controls the steam injecting means 4 to stop steam injection, and controls the pressure adjusting means 5 to release the pressure adjusting valve 51. This procedure causes the high-pressure steam in the reaction vessel to be discharged into the exhaust pipe 5b to reduce the pressure inside the reaction vessel 2.

With this procedure, bonded molecules of hydrocarbons, proteins, fats contained in livestock excrement such as chicken droppings or hog dunk or feed as digested are separated, decomposed and changes in properties of monosaccharides, amino acids, and higher fatty acids occur. The liquid containing low-molecular organic compounds is discharged through the delivery port 2b, and supplied through a liquid-solid separator 12 to a fermentation vessel for methane fermentation processing, and methane gas is extracted. Methane fermentation processing, supplied to the fermentation vessel without passing through the liquid-solid separator 12, can be performed. The use of the liquid-solid separator 12, however, is preferable because foreign matters such as pieces of plastic bags or small stones can be eliminated.

### DESCRIPTION OF EMBODIMENTS

### Embodiment 1

After treatment of chicken dropping, ammonia odor and an amount of methane gas were examined in Embodiment 1. 750kg of chicken droppings and 4550 kg of tap water, equivalent to 6 times of the chicken droppings, are charged into the reaction vessel. Hydrothermal treatments were performed with different condition, as shown in Table 1, of the temperature and pressure of the reaction vessel 2 and the number of revolution of stirring. CODcr, pH, odor of ammonia were measured and volume of generated methane gas was also measured after the liquid subjected to hydrothermal treatment was supplied to a fermentation vessel. Results are shown in Table 1. The temperature of the fermentation vessel, temperature condition of the fermentation used in the fermentation vessel, was in a range of 30 °C to 44 °C.

**Table 1**

| Items | Temperature | Pressure | Number of Stirring Revolution | Raw Material Weight | Dilution Water | Total charged Material | COD_{Cr} | pH | Ammonia Odor | Generated Gas Volume | Evaluation Results |
|---|---|---|---|---|---|---|---|---|---|---|---|
| Unit | degrees Celsius | MPa | rpm | kg | kg | kg | mg/L | After hydrothermal reaction | | m³/day | |
| Experiment 1 | 110 | 0.44 | 3 | 750 | 4,550 | 5,300 | 110,000 | 7.6 | Smelly | 69 | NG |
| Experiment 2 | 115 | 0.5 | 6 | 750 | 4,550 | 5,300 | 110,000 | 7.5 | Smelly | 69 | NG |
| Experiment 3 | 125 | 0.65 | 4 | 750 | 4,550 | 5,300 | 110,000 | 7.5 | Reduced | 88 | Fair |
| Experiment 4 | 135 | 0.76 | 4 | 750 | 4,550 | 5,300 | 110,000 | 7 | Free | 102 | Excellent |
| Experiment 5 | 140 | 0.85 | 6 | 750 | 4,550 | 5,300 | 110,000 | 7 | Free | 139 | Excellent |
| Experiment 6 | 160 | 1.15 | 5 | 750 | 4,550 | 5,300 | 110,000 | 6.8 | Free | 139 | Good |
| Experiment 7 | 170 | 1.7 | 5 | 750 | 4,550 | 5,300 | 110,000 | 6.8 | Free | 140 | Good |
| Experiment 8 | 180 | 1.8 | 5.5 | 750 | 4,550 | 5,300 | 110,000 | 5.6 | Free | 149 | Good |
| Experiment 9 | 185 | 1.9 | 4 | 750 | 4,550 | 5,300 | 110,000 | 5.6 | Free | 149 | Good |

The fuel after hydrothermal treatment preferably has features that detected ammonia is nearly detection limited, pH falls in a range from 5.6 to 7.5, and the amount of the generated gas is as much as possible. For evaluation results in Table 1, "NG" is given when ammonia odor is smelly, "Fair" is given when the ammonia odor reduces and the amount of the gas volume is larger, and "Excellent" is given when the amount of the gas volume is large and the gas is free from ammonia odor. For experiments 6 to 9, "Good" is given considering an economical viewpoint because necessary amount of steam is larger though the amount of the gas volume is large and the gas is free from ammonia odor.

As seen from Experiments 1 through 9, when the temperature of the liquid in the reaction vessel 2 is equal to 125 °C or higher and the pressure in the reaction vessel 2 is equal to 0.65 MPa or higher, then the pH value is 7.5 and the generated methane gas is equal to 88 m³/day or more, and reduction of ammonia odor is observed. In particular, when the temperature of the liquid in the reaction vessel 2 is equal to 135 °C or higher and the pressure inside the vessel is equal to 0.76 MPa or higher, the pH value falls in a range from 5.6 to 7.0 and the amount of generated methane gas is equal to 102 m³/day or more and the gas is free from ammonia odor. It is confirmed that this condition is preferable. Meanwhile, the condition where the temperature of the liquid in the vessel is equal to 160 °C or higher is also preferable because the amount of the generated gas is equal to 102 m³ or more and the gas is free from ammonia odor, but it is uneconomical due to the need of large amount of steam. In some cases the liquid is acidic with a pH value of 5.6, in
this case neutralization processing is necessary.

From a commercial viewpoint, the condition is desirable that the temperature of the liquid in the vessel 2 is in a range from 135 °C to 140 °C and the pressure inside the vessel is in a range from 0.76 MPa to 0.85 MPa, where the pH value is 7 and the amount of generated gas is in a range from 102 m³/day to 139 m³/day or more and the gas is free from ammonia odor.

### Embodiment 2

By changing the amount of water for dilution to a charged amount of chicken dropping, odor of ammonia and an amount of methane gas were examined in Embodiment 2. 750kg of chicken droppings and tap water equivalent to four (4) to twenty (20) times as much as the chicken droppings, are charged into the reaction vessel. Hydrothermal treatments were performed under different conditions, as shown in Experiments in Table 2, of the temperature ranging from 112 °C to 180 °C and pressure inside the reaction vessel 2 in a range of 0.38 MPa to 1.5 MPa and the number of revolution of stirring ranging from 3 to 6 rpm. Similar to Experiments 1 to 9 in Table 1, CODcr, pH, and ammonia odor were measured and volume of generated methane gas was also measured after the liquid subjected to hydrothermal treatment was supplied to a fermentation vessel. Results are shown in Table 2. For Experiments 10 to 18, similar to Experiments 1 to 9, temperature of the fermentation vessel, temperature condition of the fermentation used in the fermentation vessel, was in a range of 30 °C to 44 °C.

| Items | Temperature | Pressure | Number of Stirring Revolution | Raw Material Weight | Dilution Water | Total charged Material | Dilution ratio | CODcr | pH | Ammonia Odor | Generated Gas Volume | Evaluation Results |
|---|---|---|---|---|---|---|---|---|---|---|---|---|
| Unit | degrees Celsius | MPa | rpm | kg | kg | kg | | mg/L | After hydrothermal reaction | | m³/day | |
| Experiment 10 | 112 | 0.38 | 3 | 750 | 3,000 | 3,750 | 4 | 110,000 | 5.9 | Smelly | 61 | NG |
| Experiment 11 | 115 | 0.55 | 6 | 750 | 3,750 | 4,500 | 5 | 110,000 | 6 | Smelly | 68 | NG |
| Experiment 12 | 120 | 0.62 | 4 | 750 | 3,750 | 4,500 | 5 | 110,000 | 6 | Reduced | 90 | Fair |
| Experiment 13 | 138 | 0.69 | 4 | 750 | 7,500 | 8,250 | 10 | 110,000 | 6.4 | Free | 109 | Excellent |
| Experiment 14 | 151 | 0.84 | 6 | 750 | 9,000 | 9,750 | 12 | 110,000 | 6.9 | Free | 120 | Excellent |
| Experiment 15 | 155 | 0.89 | 5 | 750 | 11.250 | 12,000 | 15 | 110,000 | 6.8 | Free | 71 | Good |
| Experiment 16 | 167 | 1.08 | 5 | 750 | 15,000 | 15,750 | 20 | 110,000 | 7.1 | Free | 92 | Good |
| Experiment 17 | 173 | 1.11 | 5.5 | 750 | 15,000 | 15,750 | 20 | 110,000 | 7.1 | Free | 100 | Good |
| Experiment 18 | 180 | 1.5 | 4 | 750 | 15,000 | 15,750 | 20 | 110,000 | 7 | Free | 96 | Good |

For evaluation results in Table 2, "NG" is given when ammonia odor is smelly, "Fair" is given when the ammonia odor reduces and the amount of the gas volume is larger, and "Excellent" is given when the amount of the gas volume is large and the gas is free from ammonia odor. For experiments 15 to 18, "Good" is given considering an economical viewpoint because necessary amount of steam is larger though the amount of the gas volume is large and the gas is free from ammonia odor.

As seen from the Experiments 10 to 18, it is confirmed desirable that the amount of the tap water is five (5) to twenty (20) as much as the charge chicken droppings. The condition where the temperature of the reaction vessel 2 is equal to 155 °C or higher is also desirable because the gas is free from ammonia odor, but it is uneconomical due to the need of a large amount of steam.

### Embodiment 3

After treatment of hog dung, ammonia odor and an amount of methane gas were examined in Embodiment 3. Hydrothermal treatments were performed with different condition, as shown in Experiments 19 to 25 in Table 3, of the temperature of the liquid in the reaction vessel 2, pressure inside the reaction vessel, the amount of dilution water, and the number of revolution of stirring. CODcr after hydrothermal treatment, pH, and ammonia odor were measured and volume of generated methane gas was also measured after the liquid subjected to hydrothermal treatment was supplied to a fermentation vessel. Results are shown in Table 3. The temperature of the fermentation vessel, temperature condition of the fermentation used in the fermentation vessel, was in a range of 30 °C to 44 °C.

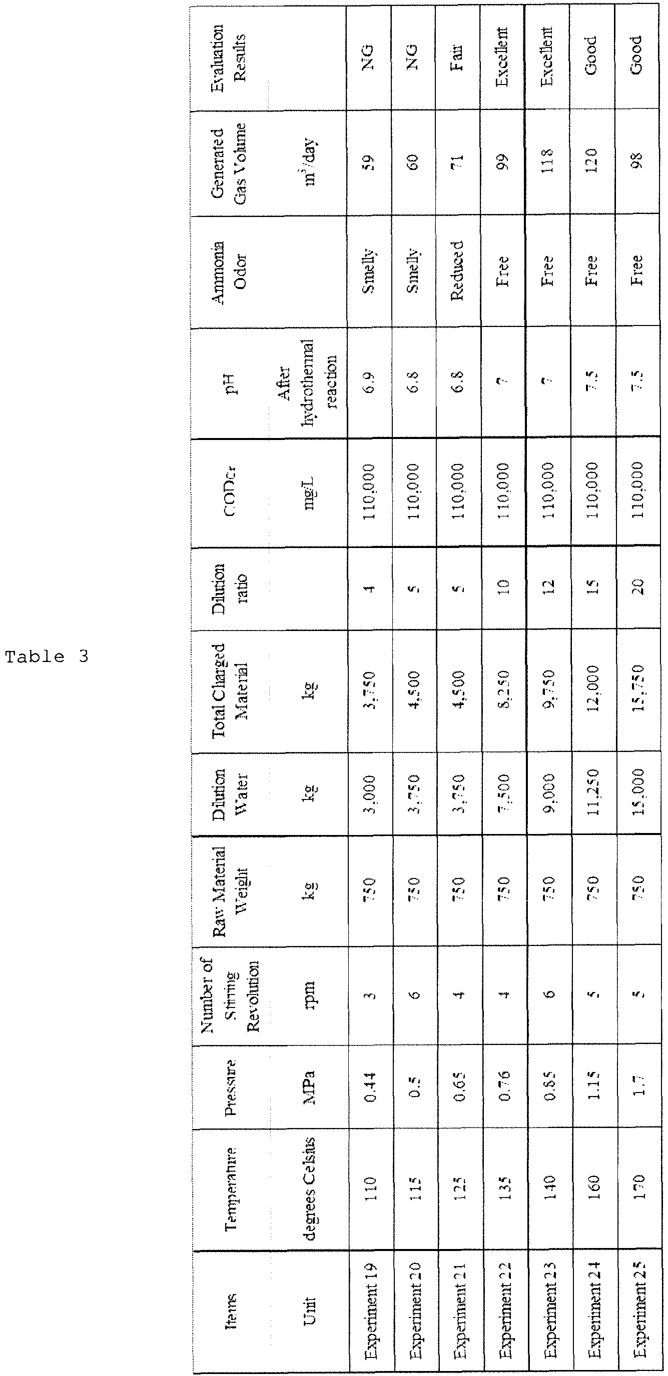

As a result, in a case where the temperature of the liquid is equal to 125 °C or higher and the pressure in the reaction vessel is equal to 0.65 MPa or higher, then the pH values falls in a range of 6.8 to 7.5, the gas is free from ammonia odor or the odor is reduced, and the amount of generated methane gas is equal to 71 m³/day or more; the processing condition was confirmed to be desirable. In particular, the condition was confirmed to be desirable that the pressure in the reaction vessel is in a range from 0.76 MPa to 0.85 MPa, the pH values falls in a range of 6.8 to 7.5, the gas is free from ammonia odor or the odor is reduced, the amount of generated methane gas is equal to 99 m³/day or more, and the temperature at the bottom of the reaction vessel is in a range of 135 °C to 140 °C. The condition where the temperature of the reaction vessel 2 is equal to 160 °C or higher is also desirable because the gas is free from ammonia odor, but it is uneconomical due to the need of a large amount of steam.

### Explanation of reference numerals

- 2: reaction vessel
- 2a: charging port
- 2b: delivery port
- 3: stirring means
- 3a: horizontal rotary shaft
- 3b: stirring blades
- 3c: driving motor
- 4: steam injecting means
- 4a: boiler
- 4b: steam supply pipe
- 5: pressure adjusting means
- 5a: pressure adjusting valve
- 5b: exhaust pipe
- 6: control means
- 7: temperature sensor
- 8: pressure sensor
- 9: silencer
- 10: cooling device
- 11: waste water treatment equipment
- 12: liquid-solid separator

## Claims

1. A fuel production method, comprising steps of:
charging livestock excrement into a reaction vessel (2) and charging water the amount of which is equal to or more than five times or equal to or less than twenty times as much as the charged livestock excrement;
injecting saturated steam having a pressure of 0.62 MPa or higher or 1.9 MPa or lower into the reaction vessel after the livestock excrement and water are charged;
low-molecularizing by decomposing the organic bonded molecules contained in the livestock excrement, while removing ammonia by stirring the contents of the reaction vessel with injecting steam on; and
stopping stirring when the temperature of a bottom portion in the reaction vessel reaches 125 °C or above.

2. The fuel production method according to claim 1, wherein the stirring is started at a time when the temperature at the bottom of the reaction vessel reaches 100 °C.

3. The fuel production method according to claim 1 or 2, comprising steps of:
charging chicken droppings as the livestock excrement into the reaction vessel and
charging water the amount of which is equal to or more than five times or equal to or less than ten times as much as the charged chicken droppings;
injecting saturated steam having a pressure of 0.62 MPa or higher or 0.84 MPa or lower into the reaction vessel after the chicken droppings and water are charged;
low-molecularizing by decomposing the organic bonded molecules contained in the chicken droppings, while removing ammonia by stirring the contents of the reaction vessel with injecting steam on; and
stopping stirring when the temperature of the bottom portion in the reaction vessel rises to a range from 125 °C to 140 °C.

4. The fuel production method according to claim 1 or 2, charging pig excrement as the livestock excrement into the reaction vessel and
charging water the amount of which is equal to or more than ten times or equal to or less than twelve times as much as the charged pig excrement;
injecting saturated steam having a pressure of 0.76 MPa or higher or 0.85 MPa or lower into the reaction vessel after the pig excrement and water are charged;
low-molecularizing by decomposing the organic bonded molecules contained in the pig excrement, while removing ammonia by stirring the contents of the reaction vessel with injecting steam on; and
stopping stirring when the temperature of the bottom portion in the reaction vessel rises to a range from 135 °C to 140 °C.

5. A fuel production apparatus (1) to be used for producing fuel from livestock excrement according to the method described in claim 1 or 2, comprising:
a reaction vessel (2) having stirring means (3) and in which the livestock excrement may be stored and treated;
a charging port (2a) provided on the reaction vessel for charging the livestock excrement and water into the reaction vessel;
a delivery port (2b) provided on the reaction vessel for leading a liquid containing low molecular weight organic compounds obtained by hydrothermal reaction treatment of the livestock excrement, the charging port and the delivery port having sealed structures with packings resistant to high temperature and high pressure of the reaction vessel when the liquid is subjected to the hydrothermal treatment;
steam injection means (4) for injecting saturated high-pressure steam into the reaction vessel in which livestock excrement is charged,
a temperature sensor (7) which measures the temperature at a bottom portion of the reaction vessel, and
control means (6) that controls injection of the saturated steam using the steam injection means to keep the pressure of the reaction vessel in a range from 0.62 MPa to 1.9 MPa, controls to start operation of the stirring means at a time when the temperature at the bottom of the reaction vessel passes 100 °C, and controls to stop operation of the stirring means at a time when the temperature at the bottom reaches 125 °C.

6. The fuel production apparatus according to claim 5, wherein the livestock excrement is chicken droppings, and wherein the control means controls injection of the saturated steam using the steam injection means to keep the pressure of the reaction vessel in a range of 0.69 MPa to 0.84 MPa, controls to start operation of the stirring means at a time when the temperature at the bottom of the reaction vessel passes 100 °C, and controls to stop operation of the stirring means at a time when the temperature at the bottom reaches a temperature in a range from 135 °C to 140 °C.

7. The fuel production apparatus according to claim 5, wherein the livestock excrement is pig excrement, and wherein the control means controls injection of the saturated steam using the steam injection means to keep the pressure of the reaction vessel in a range of 0.76 MPa to 0.85 MPa, controls to start operation of the stirring means at a time when the temperature at the bottom of the reaction vessel passes 100 °C, and controls to stop operation of the stirring means at a time when the temperature at the bottom reaches a temperature in a range from 135 °C to 140 °C.

## Patentansprüche

1. Verfahren zur Erzeugung von Kraftstoff, umfassend die folgenden Schritte:
Einfüllen von tierischen Exkrementen in ein Reaktionsgefäß (2) und Einfüllen von Wasser, dessen Menge gleich oder mehr als das Fünffache oder gleich oder weniger als das Zwanzigfache der eingefüllten tierischen Exkremente beträgt;
Einleiten von gesättigtem Dampf mit einem Druck von 0,62 MPa oder höher oder 1,9 MPa oder niedriger in das Reaktionsgefäß, nachdem die tierischen Exkremente und Wasser eingefüllt wurden;
Niedermolekularisieren durch Zersetzen der in den tierischen Exkrementen enthaltenen organisch gebundenen Moleküle unter Entfernung von Ammoniak durch Rühren des Inhalts des Reaktionsgefäßes bei fortlaufender Dampfeinleitung; und
Beenden des Rührvorgangs, wenn die Temperatur des Bodenbereichs im Reaktionsgefäß 125 °C oder mehr erreicht.

2. Verfahren zur Erzeugung von Kraftstoff nach Anspruch 1, wobei der Rührvorgang zu einem Zeitpunkt einsetzt, an dem die Temperatur am Boden des Reaktionsgefäßes 100 °C erreicht.

3. Verfahren zur Erzeugung von Kraftstoff nach Anspruch 1 oder 2, umfassend die folgenden Schritte:
Einfüllen von Hühnerkot als tierische Extremente in das Reaktionsgefäß und
Einfüllen von Wasser, dessen Menge gleich oder mehr als das Fünffache oder gleich oder weniger als das Zehnfache der Menge des eingefüllten Hühnerkots beträgt;
Einleiten von gesättigtem Dampf mit einem Druck von 0,62 MPa oder höher oder 0,84 MPa oder niedriger in das Reaktionsgefäß, nachdem der Hühnerkot und Wasser eingefüllt wurden;
Niedermolekularisieren durch Zersetzen der im Hühnerkot enthaltenen organisch gebundenen Moleküle unter Entfernung von Ammoniak durch Rühren des Inhalts des Reaktionsgefäßes bei fortlaufender Dampfeinleitung; und
Beenden des Rührvorgangs, wenn die Temperatur des Bodenbereichs im Reaktionsgefäß auf einen Bereich von 125 °C bis 140 °C ansteigt.

4. Verfahren zur Erzeugung von Kraftstoff nach Anspruch 1 oder 2,
umfassend die folgenden Schritte;
Einfüllen von Schweinekot als tierische Extremente in das Reaktionsgefäß und
Einfüllen von Wasser, dessen Menge gleich oder mehr als das Zehnfache oder gleich oder weniger als das Zwölffache der Menge des eingefüllten Schweinekots beträgt;
Einleiten von gesättigtem Dampf mit einem Druck von 0,76 MPa oder höher oder 0,85 MPa oder niedriger in das Reaktionsgefäß, nachdem der Schweinekot und Wasser eingefüllt wurden;
Niedermolekularisieren durch Zersetzen der in dem Schweinekot enthaltenen organisch gebundenen Moleküle unter Entfernung von Ammoniak durch Rühren des Inhalts des Reaktionsgefäßes bei fortlaufender Dampfeinleitung; und
Beenden des Rührvorgangs, wenn die Temperatur des Bodenbereichs im Reaktionsgefäß auf einen Bereich von 135 °C bis 140 °C ansteigt.

5. Kraftstofferzeugungsvorrichtung (1) zur Verwendung bei der Erzeugung von Kraftstoff aus tierischen Exkrementen gemäß dem in Anspruch 1 oder 2 beschriebenen Verfahren, umfassend:
ein Reaktionsgefäß (2) mit einer Rühreinrichtung (3), in dem die tierischen Exkremente gelagert und behandelt werden können;
eine Einfüllöffnung (2a), die an dem Reaktionsgefäß vorgesehen ist, um die tierischen Exkremente und Wasser in das Reaktionsgefäß einzufüllen;
eine Abgabeöffnung (2b), die an dem Reaktionsgefäß vorgesehen ist, um eine Flüssigkeit zu führen, die durch hydrothermale Reaktionsbehandlung der tierischen Exkremente gewonnene, organische Verbindungen mit niedrigem Molekulargewicht enthält, wobei die Einfüllöffnung und die Abgabeöffnung abgedichtete Strukturen mit hochtemperatur- und hochdruckbeständigen Packungen für das Reaktionsgefäß umfassen, wenn die Flüssigkeit der hydrothermalen Behandlung unterzogen wird;
eine Dampfeinleiteinrichtung (4) zum Einleiten von gesättigtem Hochdruckdampf in das Reaktionsgefäß, in das tierische Exkremente eingefüllt sind,
einen Temperatursensor (7), der die Temperatur an einem Bodenbereich des Reaktionsgefäßes misst, und
eine Steuereinrichtung (6), die die Einleitung von gesättigtem Dampf unter Verwendung der Dampfeinleiteinrichtung steuert, um den Druck des Reaktionsgefäßes in einem Bereich von 0,62 MPa bis 1,9 MPa zu halten, den Betrieb der Rühreinrichtung zu einem Zeitpunkt startet, an dem die Temperatur am Boden des Reaktionsgefäßes 100 °C überschreitet, und den Betrieb der Rühreinrichtung zu einem Zeitpunkt beendet, an dem die Temperatur am Boden 125 °C erreicht.

6. Kraftstofferzeugungsvorrichtung nach Anspruch 5, wobei es sich bei den tierischen Exkrementen um Hühnerkot handelt und wobei die Steuereinrichtung die Einleitung von gesättigtem Dampf unter Verwendung der Dampfeinleiteinrichtung steuert, um den Druck des Reaktionsgefäßes in einem Bereich von 0,69 MPa bis 0,84 MPa zu halten, den Betrieb der Rühreinrichtung zu einem Zeitpunkt startet, an dem die Temperatur am Boden des Reaktionsgefäßes 100 °C überschreitet, und den Betrieb der Rühreinrichtung zu einem Zeitpunkt beendet, an dem die Temperatur am Boden eine Temperatur in einem Bereich von 135 °C bis 140 °C erreicht.

7. Kraftstofferzeugungsvorrichtung nach Anspruch 5, wobei es sich bei den tierischen Exkrementen um Schweinekot handelt und wobei die Steuereinrichtung die Einleitung von gesättigtem Dampf unter Verwendung der Dampfeinleiteinrichtung steuert, um den Druck des Reaktionsgefäßes in einem Bereich von 0,76 MPa bis 0,85 MPa zu halten, den Betrieb der Rühreinrichtung zu einem Zeitpunkt startet, an dem die Temperatur am Boden des Reaktionsgefäßes 100 °C überschreitet, und den Betrieb der Rühreinrichtung zu einem Zeitpunkt beendet, an dem die Temperatur am Boden eine Temperatur in einem Bereich von 135 °C bis 140 °C erreicht.

## Revendications

1. Procédé de production de combustible, comprenant les étapes de :
chargement d'excréments de cheptel dans un récipient réactionnel (2) et chargement d'eau en une quantité allant de cinq fois ou plus à vingt fois ou moins celle des excréments de cheptel chargés ;
injection de vapeur saturée ayant une pression de 0,62 MPa ou plus et 1,9 MPa ou moins dans le récipient réactionnel après que les excréments de cheptel et l'eau ont été chargés ;
réduction de la masse moléculaire par décomposition des molécules liées organiques contenues dans les excréments de cheptel, tout en éliminant l'ammoniac par agitation du contenu du récipient réactionnel avec l'injection de vapeur en cours ; et
arrêt de l'agitation quand la température de la portion inférieure du récipient réactionnel atteint 125°C ou plus.

2. Procédé de production de combustible selon la revendication 1, dans lequel l'agitation est démarrée au moment où la température au fond du récipient réactionnel atteint 100°C.

3. Procédé de production de combustible selon la revendication 1 ou 2, comprenant les étapes de :
chargement de fientes de poulets en tant qu'excréments de cheptel dans le récipient réactionnel et
chargement d'eau en une quantité allant de cinq fois ou plus à dix fois ou moins celle des fientes de poulets chargées ;
injection de vapeur saturée ayant une pression de 0,62 MPa ou plus ou 0,84 MPa ou moins dans le récipient réactionnel après que les fientes de poulets et l'eau ont été chargées ;
réduction de la masse moléculaire par décomposition des molécules liées organiques contenues dans les fientes de poulets, tout en éliminant l'ammoniac par agitation du contenu du récipient réactionnel avec l'injection de vapeur en cours ; et
arrêt de l'agitation quand la température de la portion inférieure du récipient réactionnel atteint une plage allant de 125°C à 140°C.

4. Procédé de production de combustible selon la revendication 1 ou 2, comprenant les étapes de :
chargement d'excréments de cochons en tant qu'excréments de cheptel dans le récipient réactionnel et
chargement d'eau en une quantité allant de dix fois ou plus à douze fois ou moins celle des excréments de cochons chargés ;
injection de vapeur saturée ayant une pression de 0,76 MPa ou plus ou 0,85 MPa ou moins dans le récipient réactionnel après que les excréments de cochons et l'eau ont été chargés ;
réduction de la masse moléculaire par décomposition des molécules liées organiques contenues dans les excréments de cochons, tout en éliminant l'ammoniac par agitation du contenu du récipient réactionnel avec l'injection de vapeur en cours ; et
arrêt de l'agitation quand la température de la portion inférieure du récipient réactionnel atteint une plage allant de 135°C à 140°C.

5. Dispositif de production de combustible (1) destiné à être utilisé pour produire du combustible à partir d'excréments de cheptel selon le procédé décrit dans la revendication 1 ou 2, comprenant :
un récipient réactionnel (2) ayant un moyen d'agitation (3) et dans lequel les excréments de cheptel peuvent être stockés et traités ;
un orifice de chargement (2a) placé sur le récipient réactionnel pour charger les excréments de cheptel et de l'eau dans le récipient réactionnel ;
un orifice de délivrance (2b) placé sur le récipient réactionnel pour guider un liquide contenant des composés organiques de faible masse moléculaire obtenus par un traitement de réaction hydrothermal des excréments de cheptel, l'orifice de chargement et l'orifice de délivrance ayant des structures scellées avec des garnitures résistant à la température élevée et à la pression élevée du récipient réactionnel quand le liquide est soumis au traitement hydrothermal ;
un moyen d'injection de vapeur (4) pour injecter une vapeur haute pression saturée dans le récipient réactionnel dans lequel les excréments de cheptel sont chargés,
un capteur de température (7) qui mesure la température au niveau d'une portion de fond du récipient réactionnel, et
un moyen de commande (6) qui commande l'injection de la vapeur saturée utilisant le moyen d'injection de vapeur pour maintenir la pression du récipient réactionnel dans la plage allant de 0,62 MPa à 1,9 MPa, commande le lancement du fonctionnement du moyen d'agitation au moment où la température au fond du récipient réactionnel passe 100°C, et commande l'arrêt du fonctionnement du moyen d'agitation au moment où la température au fond atteint 125°C.

6. Dispositif de production de combustible selon la revendication 5, dans lequel les excréments de cheptel sont des fientes de poulets, et dans lequel le moyen de commande commande l'injection de la vapeur saturée utilisant le moyen d'injection de vapeur pour maintenir la pression du récipient réactionnel dans la plage allant de 0,69 MPa à 0,84 MPa, commande le lancement du fonctionnement du moyen d'agitation au moment où la température au fond du récipient réactionnel passe 100°C, et commande l'arrêt du fonctionnement du moyen d'agitation au moment où la température au fond atteint une température située dans la plage allant de 135°C à 140°C.

7. Dispositif de production de combustible selon la revendication 5, dans lequel les excréments de cheptel sont des excréments de cochons, et dans lequel le moyen de commande commande l'injection de la vapeur saturée utilisant le moyen d'injection de vapeur pour maintenir la pression du récipient réactionnel dans la plage allant de 0,76 MPa à 0,85 MPa, commande le lancement du fonctionnement du moyen d'agitation au moment où la température au fond du récipient réactionnel passe 100°C, et commande l'arrêt du fonctionnement du moyen d'agitation au moment où la température au fond atteint une température située dans la plage allant de 135°C à 140°C.
